Europäisches Patentamt

European Patent Office

Office européen des brevets

(11) Veröffentlichungsnummer : **0 093 230**
**B1**

(12) **EUROPÄISCHE PATENTSCHRIFT**

(45) Veröffentlichungstag der Patentschrift :
28.05.86

(51) Int. Cl.⁴ : **A 61 F  2/32**

(21) Anmeldenummer : **83101097.0**

(22) Anmeldetag : **05.02.83**

(54) **Knochenimplantat, insbesondere femorale Hüftgelenkprothese.**

(30) Priorität : **03.05.82 DE 3216538**

(43) Veröffentlichungstag der Anmeldung :
**09.11.83 Patentblatt 83/45**

(45) Bekanntmachung des Hinweises auf die Patenterteilung : **28.05.86 Patentblatt 86/22**

(84) Benannte Vertragsstaaten :
**CH DE FR GB IT LI SE**

(56) Entgegenhaltungen :
**EP-A- 0 065 481**
**DE-A- 2 839 093**
**FR-A- 2 325 355**

(73) Patentinhaber : **Waldemar Link GmbH & Co**
**Barkhausenweg 10**
**D-2000 Hamburg 63 (DE)**

(72) Erfinder : **Link, Helmut D.**
**Wildstieg 14**
**D-2000 Hamburg 65 (DE)**
Erfinder : **Keller, Arnold**
**An der Naher Furth 5**
**D-2061 Kayhude (DE)**

(74) Vertreter : **Glawe, Delfs, Moll & Partner Patentan-**
**wälte**
**Postfach 26 01 62 Liebherrstrasse 20**
**D-8000 München 26 (DE)**

Anmerkung : Innerhalb von neun Monaten nach der Bekanntmachung des Hinweises auf die Erteilung des
europäischen Patents im Europäischen Patentblatt kann jedermann beim Europäischen Patentamt gegen das erteilte
europäische Patent Einspruch einlegen. Der Einspruch ist schriftlich einzureichen und zu begründen. Er gilt erst als
eingelegt, wenn die Einspruchsgebühr entrichtet worden ist (Art. 99(1) Europäisches Patentübereinkommen).

**Beschreibung**

Die Erfindung bezieht sich auf ein Knochenimplantat, insbesondere eine femorale Hüftgelenkprothese, mit einem Kopfteil, einem im Knochen zu verankernden Schaft und einer den Schaft kopfseitig abschließenden Halsauflage.

Muß die Prothese im Rahmen einer Reoperation entfernt werden, ist es häufig notwendig, den Verbund zwischen dem Prothesenschaft und dem Knochengewebe bzw. dem Knochenzement mittels eines Knochenmeißels zu lösen. Dies kann aber auf Schwierigkeiten stoßen, wenn der Prothesenschaft kopfseitig durch eine sog. Halsauflage abgeschlossen ist. Dies ist ein teller- oder kragenförmiger, im allgemeinen umlaufender Vorsprung, der sich an der proximalen Resektionsfläche des Knochens abstützt. Manche Operateure sind daher zur Verwendung von Prothesen ohne Halsauflage übergegangen, obwohl das Zusammenwirken der Halsauflage mit der Resektionsfläche infolge von deren günstiger Lage im Verhältnis zur Kraftrichtung zweifellos einem wertvollen Beitrag zur gesamten Kraftübertragung zu leisten vermag.

Der Erfindung liegt daher die Aufgabe zugrunde, ein Knochenimplantat der eingangs genannten Art zu schaffen, die leichter entfernt werden kann, ohne daß deshalb auf die Halsauflage verzichtet werden müßte.

Die erfindungsgemäße Lösung besteht darin, daß die Halsauflage bei eingesetzter Prothese einsetzbar und lösbar ist.

Muß die Prothese entfernt werden, so wird zunächst die Halsauflage gelöst. Dadurch gewinnt der Operateur leichten Zugang zum Schaft und kann mit dem Knochenmeißel den Verbund zwischen Prothese und Knochen lösen.

Es ist zwar eine Prothesenanordnung bekannt (FR-A-2 325 355), bei welcher ein mit der Halsauflage zusammenwirkender Teil mit dem Prothesenschaft nicht fest verbunden ist, so daß er von diesem lösbar ist. Dabei handelt es sich um einen an einer Schmalseite U-förmig geöffneten, ovalen Ring mit winkelförmigem Querschnitt, der mit einem Querschnittschenkel in die Öffnung der Markhöhle eingesetzt und mit dem anderen Querschnittschenkel auf der Resektionsfläche des Oberschenkelknochens aufliegt. Er besteht aus elastischem Material und hat den Zweck, den Schaft in der Markhöhle einzuklemmen und die fest mit dem Schaft verbundene Halsauflage der Prothese elastisch und schockdämpfend abzustützen. Weder ist bei der bekannten Anordnung eine lösbare Halsauflage vorhanden, noch wird die Reoperation erleichtert.

Da der Oberschenkelkopf gegenüber der Richtung des Oberschenkelknochens seitlich versetzt liegt, ist der Oberschenkelknochen im Stand einer Biegebeanspruchung unterworfen, die lateral eine Zugbeanspruchung und medial eine Druckbeanspruchung im Knochen hervorruft. Wenn Kopf und Hals der Oberschenkelknochen durch eine Schaftprothese ersetzt werden, deren Schaft im Markhohlraum des Knochens verankert ist, so ändert sich der Kraftverlauf im Knochen fundamental. Insbesondere wird die laterale Seite von den Zugkräften völlig entlastet, während medial starke Druckkräfte vom Prothesenschaft auf das Knochengewebe zu übertragen sind. Sowohl die laterale Entlastung des Knochens als auch eine evtl. Überlastung im medialen Bereich können zu Degenerationserscheinungen und nachfolgender Lockerung der Prothese führen.

Die Erfindung hat erkannt, daß sich der laterale Knochenbereich, insbesondere auch am prothesenseitigen Ende des Knochens, dadurch an der Kraftübertragung beteiligen läßt, daß die die Prothese auf der dem großen Trochanter abgewandten Seite umfassende Halsauflage einen Anker zur Befestigung am großen Trochanter aufweist. Die am großen Trochanter fixierte Halsauflage wird dadurch zu einem Element, das die Prothese gegenüber denjenigen Kräften, die sie nach medial zu drängen versuchen, abstützt. Einerseits wird dadurch die mediale Knochenseite entlastet, während andererseits die Entlastungskräfte von dem großen Trochanter und damit von der daran anschließenden lateralen Knochenseite aufzunehmen sind.

Die Verankerung der Halsauflage am großen Trochanter kann jede geeignete Gestalt annehmen. Besonders vorteilhaft ist es, die Halsauflage haken- oder hufeisenförmig auszuführen und den Anker an dem freien Hakenende bzw. den Hufeisenenden angreifen zu lassen.

Es ist in manchen Fällen nicht erforderlich, die Halsauflage gegenüber der Prothese in deren Längsrichtung zu fixieren. Auch bei fehlender Fixierung stützt die Halsauflage die Prothese nicht nur gegenüber nach medial drängenden Kräften ab, sondern überträgt sie auch einen Teil der vertikalen Kräfte auf die Resektionsfläche des Knochens. Jedoch ist es meist zweckmäßig, die Halsauflage und den dafür an der Prothese vorgesehenen Sitz mit zusammenwirkenden, quer zur Schaft- bzw. Halsrichtung verlaufenden Rippen und Nuten zu versehen. Nach dem Einsetzen der Prothese in den Röhrenknochen wird die Halsauflage an der Prothese fixiert, indem sie quer zur Prothesenrichtung und übereinstimmend mit der Richtung der Rippen und Nuten an die Prothese angeschoben wird, so daß sie sie beiderseits umgreift. Anschließend wird sie am großen Trochanter verankert.

Die Erfindung wird im folgenden näher unter Bezugnahme auf die Zeichnung erläutert, die ein vorteilhaftes Ausführungsbeispiel veranschaulicht. Darin zeigen :

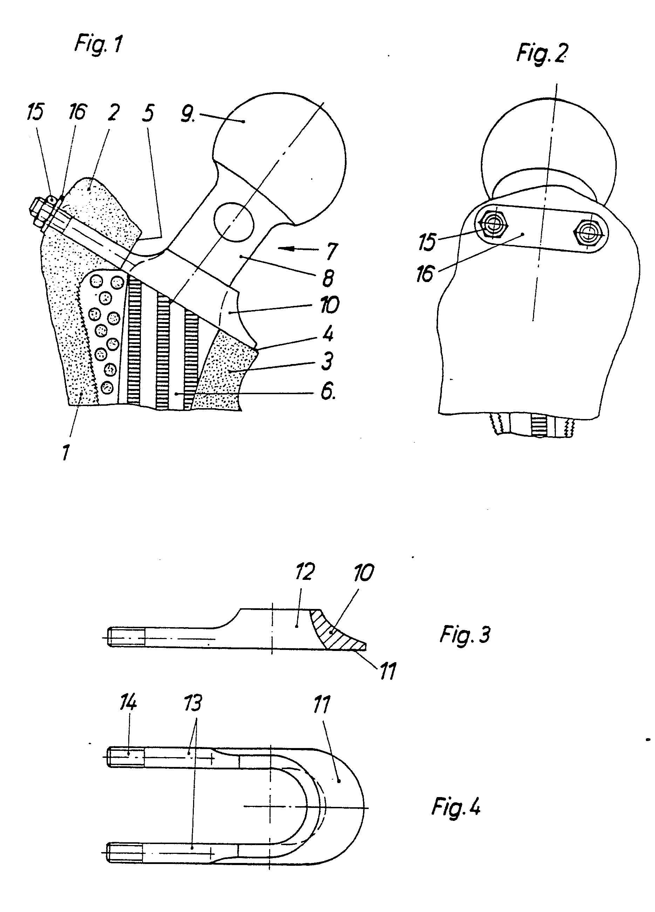

Figur 1 einen Lateral-Medial-Schnitt durch das obere Ende eines mit einer Schaftprothese versehenen Oberschenkelknochens,

Figur 2 eine Ansicht derselben Teile von lateral,

Figur 3 einen Längsschnitt durch die Halsauflage und

Figur 4 einen Draufsicht auf die Halsauflage.

In der Schnittansicht ist punktiert der Oberschenkelknochen 1 angedeutet, dessen großen Trochanter man bei 2 erkennt, während mit 3 die medial gegenüberliegenden Knochenbereiche bezeichnet sind. Längs der Resektionsfläche 4 und 5 ist der Oberschenkelhals mit dem Oberschenkelkopf abgetrennt.

In die Markhöhle ist der Schaft 6 der Prothese 7 eingesetzt, die am Hals 8 den Kopf 9 trägt. Am Übergang vom Hals 8 zum Schaft 6 befindet sich die Halsauflage 10, die mit ihrer Unterfläche 11 auf der Resektionsfläche 4 zumindest medial, aber bis zu einem gewissen Grade auch ventral und dorsal, aufliegt. Ihre Innenflächen 12 sind der Außengestalt des sie aufnehmenden Sitzes der Prothese angepaßt. Die Halsauflage ist hufeisenförmig ausgeführt, wobei an die Hufeisenenden zwei Bolzen 13 angeschließen, die durch Bohrungen des großen Trochanter hindurchgeführt sind und am Ende ein Gewinde 14 tragen, das die Schraubverankerung 15 über eine Druckplatte 16 ermöglicht. Die Verankerung ist so eingestellt, daß an der Aufnahme der an der Prothese nach medial drängenden Kräften durch den Knochen sowohl der große Trochanter 2 über die Verankerung 15, 16 als auch der mediale Knochenbereich 3 über den direkten Kontakt mit dem anliegenden Teil des Schafts 6 beteiligt wird.

Die Sitzfläche 12 der Halsauflage und die zugehörige Sitzfläche der Prothese können mit zusammenwirkenden Rippen und Nuten versehen sein, die etwa in Richtung der Bolzen 13 verlaufen, so daß sie durch das seitliche Aufschieben der Halsauflage auf den zugehörigen Sitz der Prothese in Eingriff miteinander kommen und die in Längsrichtung der Prothese auftretenden Kräfte zu übertragen vermögen. Im Falle einer Reoperation kann die Halsauflage abgenommen werden, bevor der Operateur den Verbund zwischen Knochen und Prothesenschaft zu lösen beginnt.

Die Prothese eignet sich auch für diejenigen Einsatzfälle, in denen auf eine Halsauflage aus anderen als den beschriebenen Gründen gänzlich verzichtet werden soll.

## Patentansprüche

1. Knochenimplantat, insbesondere Hüftgelenkprothese, mit einem Kopfteil (9), einem im Knochen (1) zu verankernden Schaft und einer den Schaft (6) kopfseitig abschließenden Halsauflage (10), dadurch gekennzeichnet, daß die Halsauflage (10) bei eingesetzter Prothese (7) einsetzbar und lösbar ist.

2. Knochenimplantat nach Anspruch 1, dadurch gekennzeichnet, daß die Halsauflage (10) einen Anker (13, 14, 15, 16) zur Befestigung am großen Trochanter (2) aufweist und die Prothese (7) auf der gegenüberliegenden Seite umfaßt.

3. Knochenimplantat nach Anspruch 2, dadurch gekennzeichnet, daß die Halsauflage (10) haken- oder hufeisenförmig ausgeführt ist und der Anker (13, 14, 15, 16) an dem freien Haken- bzw. den Hufeisenenden angreift.

4. Knochenimplantat nach einem der Ansprüche 1 bis 3, dadurch gekennzeichnet, daß die Halsauflage (10) und der dafür an der Prothese (7) vorgesehene Sitz mit zusammenwirkenden, quer zur Schaft- bzw. Halsrichtung verlaufenden Rippen und Nuten versehen sind.

## Claims

1. A bone implant, in particular a hip joint prosthesis, having a head part (9), a shaft to be anchored in the bone (1) and a collar support (10) terminating the shaft (6) at the head end, characterised in that the collar support (10) can be fitted and detached when the prosthesis (7) is fitted.

2. A bone implant according to Claim 1, characterised in that the collar support (10) has an anchoring means (13, 14, 15, 16) which is to be secured to the major trochanter (2) and, on the opposite side, embraces the prosthesis (7).

3. A bone implant according to Claim 2, characterised in that the collar support (10) is of a hook or horseshoe-shaped design and the anchoring means (13, 14, 15, 16) is applied at the free end of the hook or horsehoe.

4. A bone implant according to any Claims 1 to 3, characterised in that the collar support (10) and the seating provided therefor on the prosthesis (7) are provided with cooperating ribs and grooves extending transversely to the shaft and neck direction respectively.

## Revendications

1. Implant dans l'os, en particulier prothèse fémorale de hanche, comprenant une partie tête (9), une tige destinée à être scellée dans l'os (1) et un support de col (10) qui termine la tige (6) du côté tête, caractérisé en ce que le support de col (10) peut être mis en place et retiré alors que la prothèse (7) est en place.

2. Implant dans l'os selon la revendication 1, caractérisé en ce que le support de col (10) comporte des moyens d'ancrage (13, 14, 15, 16) pour sa fixation au grand trochanter (2) et entoure la prothèse (7) du côté opposé.

3. Implant dans l'os selon la revendication 2, caractérisé en ce que le support de col (10) est réalisée sous forme recourbée en crochet ou en fer à cheval et en ce que les moyens d'ancrage (13, 14, 15, 16) saisissent l'extrémité libre du crochet ou fer à cheval.

4. Implant dans l'os selon l'une quelconque des revendications 1 à 3, caractérisé en ce que le support de col (10) et le siège qui est prévu pour celui-ci sur la prothèse (7) sont munis de nervures et rainures qui s'étendent dans une direction perpendiculaire à celle de la tige ou du col et qui coopèrent.

Fig. 1

15  16  2  5  9.

7

8

10

4

3

6.

1

Fig. 2

15
16

12  10

Fig. 3

11

14  13  11

Fig. 4